# EUROPEAN PATENT APPLICATION

(11) **EP 2 499 986 A1**
(43) Date of publication of application: **19.09.2012**
(21) Application number: 11158771.3
(22) Date of filing: 18.03.2011
(51) Int. Cl.: A61B 19/00, F21V 21/26, A61G 7/10, F21S 8/00, A61B 6/00

(54) **Utility system for use in a surgical operating room**

(71) Applicant: Nordic Medical Supply A/S, 2750 Ballerup (DK)
(72) Inventor: Bodi, Svend Erik, 4632, Bjæverskov (DK)
(74) Representative: Plougmann & Vingtoft A/S

(57) **Abstract**

A utility system for use in a surgical operating room comprises a first pair of parallel rails (X1, X2) and a second pair of parallel rails (Y1, Y2) extending transversally to, and supported by, the first pair of rails and movable along the first pair of rails. A support structure (13) is supported by the second pair of rails and is movable along the second pair of rails. A utility module (17, 19, 20) secured to the support structure (13). If the utility module is a surgical lamp with a suspension system comprising articulated rigid members, a reduced number of rigid members or shorter ones are sufficient to cover the same field and with the required flexibility. Other utility modules such as anaesthesia systems, X-ray and other imaging systems, a video camera, displays for displaying diagnostic and other images, ECG and other data may also be supported.

## Description

### FIELD OF THE INVENTION

The invention relates to utility systems for use in a surgical operating room and in particular to utility systems that according to need can be moved and arranged in the operating room before, during and after a surgical operation. Utility systems comprise surgical lamps, anaesthesia systems, X-ray and other imaging systems such as a video camera, displays for displaying diagnostic and other images, ECG and other data.

### BACKGROUND OF THE INVENTION

Utility systems for use in surgical operating rooms often need to be moved and rearranged. In particular during surgical operations moving and rearranging e.g. a surgical lamp must be easy for the surgeon and his/her assistants. Surgical lamps are often suspended from the ceiling where they are fixedly mounted centrally above an operating table. I order to ensure that the lamp can be used at the extremities of the operating table and to provide adequate illumination at all possible locations the lamp must be movable within wide limits. Surgical lamps have a suspension system with a plurality of rigid members interconnected by joints allowing this. Movability within wide limits is obtained by at least some of the rigid members being fairly long, and flexibility is ensured by a number of joints required for the desired flexibility. The suspension system must be rigid and stable, and the ceiling mounting must carry the load of the lamp and its suspension system and resist the fairly large torque that results when the lamp is used in an extreme position.

EP 2 136 125 B1 discloses an example of a surgical lamp where an articulated suspension system includes six rigid members and seven interconnecting joints between the lamp and the rigid mounting at the ceiling. Other surgical lamps are known having a suspension system with more articulated rigid members.

### SUMMARY OF THE INVENTION

The invention provides a utility system for use in a surgical operating room with a suspension system for utility modules that gives the desired flexibility. The utility system of the invention includes a support structure that is carried by a system of two pairs of parallel rails transversal to each other whereby the support structure can be moved virtually to any position in the operating room.

If the utility module is a surgical lamp with a suspension system comprising articulated rigid members, a reduced number of rigid members or shorter ones are sufficient to cover the same field and with the required flexibility.

Other utility modules such as an anaesthesia module, X-ray and other imaging systems such as a video camera, displays for displaying diagnostic and other images, ECG and other data may also be supported. Anaesthesia modules are known in the art and are used for administering anaesthetics and/or sedatives, ventilating the patient and monitoring, and possibly supporting, vital physiological functions etc. during surgery.

The rails are preferably mounted at opposed walls or the ceiling of the operating room or carried by columns supported by the floor, or they can be suspended from the ceiling of the operating room. Hereby cabling etc. to the utility modules will not take up place on the floor.

### BRIEF DESCRIPTION OF THE DRAWINGS

Figure 1 shows schematically a plan view of a utility system according to the invention,
Figure 2 shows the utility system in figure 1 along the line II-II with a utility suspended module, and
Figure 3 shows the utility system in figure 1 along the line II-II with a surgical lamp suspended.

### DETAILED DESCRIPTION OF THE INVENTION

In the figures is shown a first pair of parallel rails X1, X2 which preferably are fixedly mounted horizontally at opposite walls (not shown) of a surgical operating room.

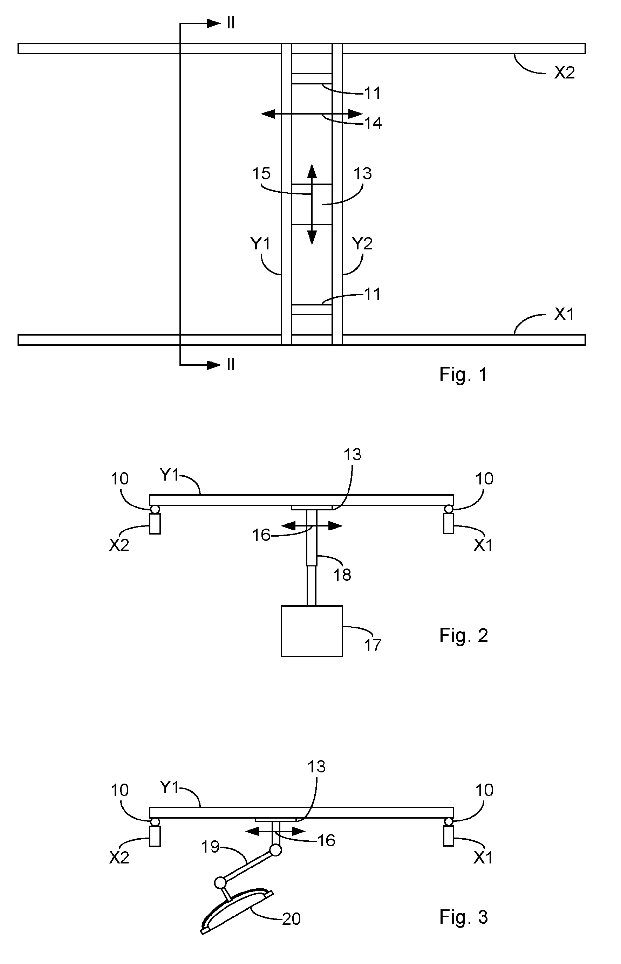

A second pair of rigidly interconnected, parallel rails Y1, Y2 are supported by the first pair of rails X1, X2 and extend horizontally and transversely, preferably perpendicular, to the first pair of rails. Wheels or rollers 10 are provided between the first and second pair of rails whereby the second pair of rails Y1, Y2 is movable along the first pair of rails X1, X2. The second pair of rails is rigidly interconnected by rigid fixing means such as bars 11 secured to each one of the second pair of rails. The second pair of rails Y1, Y2 thereby is movable as one unit along the first pair of rails X1, X2 as indicated by the arrow 14 in figure 1.

A support structure 13 is supported by the second pair of rails Y1, Y2 and wheels or rollers (not shown) are provided between the second pair of rails and the support structure 13 whereby the support structure is movable along the second pair of rails as indicated by the arrow 15 in figure 1 and the arrow 16 in figure 2.

In figure 2 is schematically illustrated a generalised utility module 17 secured to the support structure 13 and suspended therefrom. The support structure 13 can be moved in two dimensions together with the utility module 17 around to any location in a surgical operating room within the limits defined by the suspension system of the first and the second pairs of rails. The suspension carrying the utility module 17 can be a rigid member or a vertically adjustable member such as a telescopic elevation system 18.

In figure 3 is illustrated a surgical lamp 20 with a suspension system 19 secured to the support structure 13 and suspended therefrom. The support structure 13 with the utility module 17 can be moved around to any location in a surgical operating room to any position where light is desired. The entire system with the two pairs of rails X1, X2, Y1, Y2, the support structure 13 and the surgical lamp 20 and its suspension system 19 can be seen as a surgical lamp system where the two pairs of rails provide mobility in two dimensions, and since the articulated suspension system 19 of the surgical lamp 20 secured to the support structure can be moved to virtually any desired position in two dimensions, the articulated suspension system 19 of the surgical lamp 20 will mainly need to provide movability in the third dimension, i.e. vertically, by being elevated or lowered, and only limited further movement in the horizontal directions, if any, will be required by the suspension system 19. In some cases a telescopic elevation system as in figure 2 can therefore replace the articulated suspension system 19, and possibly only the lamp head 20 needs be articulated.

Each one of the rails X1, X2, Y1 and Y2 can also be used for supporting one or more utility modules to be movable along the rail.

## Claims

1. A utility system for use in a surgical operating room, the system comprising
• a first pair of parallel rails (X1, X2),
• a second pair of parallel rails (Y1, Y2) extending transversally to, and supported by, the first pair of rails (X1, X2) and movable along the first pair of rails (X1, X2),
• a support structure (13) supported by the second pair of rails (Y1, Y2) and movable along the second pair of rails (Y1, Y2), and
• a utility module (17, 19, 20) secured to the support structure (13).

2. The utility system of claim 1 wherein
• the utility module (17, 19, 20) is secured to the support structure (13) by means of a suspension structure allowing the utility module to be elevated and lowered.

3. The utility system of claim 2 wherein
• the suspension structure includes a plurality of articulated members forming an articulated structure.

4. The utility system of claim 3 wherein
• the articulated structure allows the utility module to be moved in three dimensions and to be held in a plurality of positions.

5. The utility system of any one of the preceding claims wherein
• the utility module includes a surgical lamp (19, 20).

6. The utility system of any one of the preceding claims wherein
• the utility module includes an anaesthesia module.

7. Use of a utility system of any one of the preceding claims in a surgical operating room.
